(19) Europäisches Patentamt
European Patent Office
Office européen des brevets

(11) Publication number: **0 189 144 B1**

# (12) EUROPEAN PATENT SPECIFICATION

(45) Date of publication of the patent specification: **12.04.89**

(51) Int. Cl.⁴: **C 07 D 309/18**, A 61 K 7/46, C 11 B 9/00

(21) Application number: **86100594.0**

(22) Date of filing: **17.01.86**

(54) **Pyran derivatives, process for the preparation thereof, and perfume composition containing the same.**

(30) Priority: **23.01.85 JP 10516/85**

(43) Date of publication of application: **30.07.86 Bulletin 86/31**

(45) Publication of the grant of the patent: **12.04.89 Bulletin 89/15**

(84) Designated Contracting States: **CH DE FR GB LI NL**

(56) References cited:
**WO-A-81/00204**
**CH-A- 604 560**
**DE-A- 1 908 756**
**US-A- 2 452 977**

**The file contains technical information submitted after the application was filed and not included in this specification**

(73) Proprietor: **Kao Corporation, 14-10, Nihonbashi Kayabacho 1-chome, Chuo-Ku Tokyo 103 (JP)**

(72) Inventor: **Takada, Hiroshi, 706-139, Zenmyoji Wakayama-shi, Wakayama-ken (JP)**
Inventor: **Masuda, Shin'ichi, 1130, Nishihama Wakayama-shi, Wakayama-ken (JP)**
Inventor: **Nakajima, Motoki, 1-22-6, Miyashirodai Miyashiro-cho, Minami-Saitama-gun Saitama-ken (JP)**

(74) Representative: **Kraus, Walter, Dr. et al, Patentanwälte Kraus, Weisert & Partner Thomas-Wimmer-Ring 15, D-8000 München 22 (DE)**

EP 0 189 144 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an isomeric mixture of a novel pyran derivative represented by the following general formula (I):

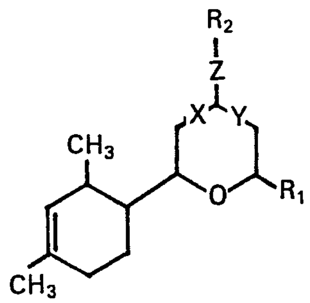

(I)

wherein $R_1$ stands for H or $CH_3$ and $R_2$ for $CH_2$ or $CH_3$, one of the bonds X, Y, and Z is a double bond and the other two are single bonds.

It has been known that 2,4-dimethyl-3-cyclohexene-1-carboxyaldehyde, represented by the following formula (II):

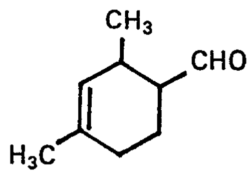

(II)

has a strong leaf-like green note (see Perfume and Flavor Chemicals I by Steffen Arctander No. 996).

However, said compound of the above formula (II) was disadvantageous in stability because it contained a formyl group.

CH-A-604 560 discloses, interalia a pyran derivative represented by the following formula:

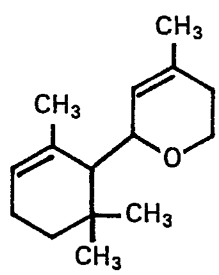

It is mentioned in the specification that the pyran derivatives have a floral note which is a little green, sometimes slightly rose note and in some cases the note of neroli or bergamot.

WO-A-8 100 204 discloses a pyran derivative represented by the following formula:

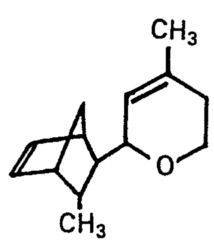

It is mentioned in the specification that this norbornyl-substituted pyran has green, woody, sweet, fruity, aldehydic odor.

The inventors have accomplished the present invention as the result of intensive studies to overcome the problem described above, based on the finding that an isomeric mixture of a pyran derivative represented by the above formula (I), obtained by reacting said compound (II) with a compound represented by the following general formula (III):

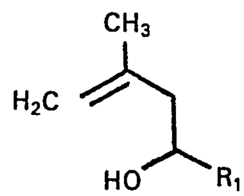

(III)

wherein $R_1$ is as defined above, is useful as a raw material for the preparation of perfume compositions because said compound has not only excellent stability but persistent and fresh tomato leaf green note.

Accordingly, the present invention provides an isomeric mixture of a novel pyran derivative represented by the general formula (I), a process for the preparation of said pyran derivative mixture, and a perfume composition containing said pyran derivative mixture.

The pyran derivative (I) of the present invention has the following three isomers, which can be distinguished by the position of the double bond:

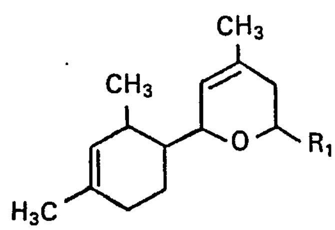

(Ia)

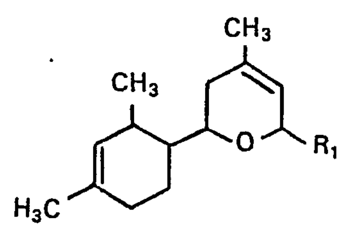

(Ib)

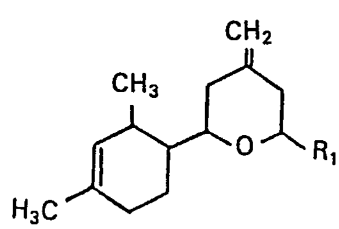

(Ic)

wherein $R_1$ is as defined above.

The present pyran derivative mixture can be prepared by reacting 2,4-dimethyl-3-cyclohexene-1-carboxyaldehyde (II) with the compound (III) in the presence of an acid catalyst according to, for example, the following reaction formula:

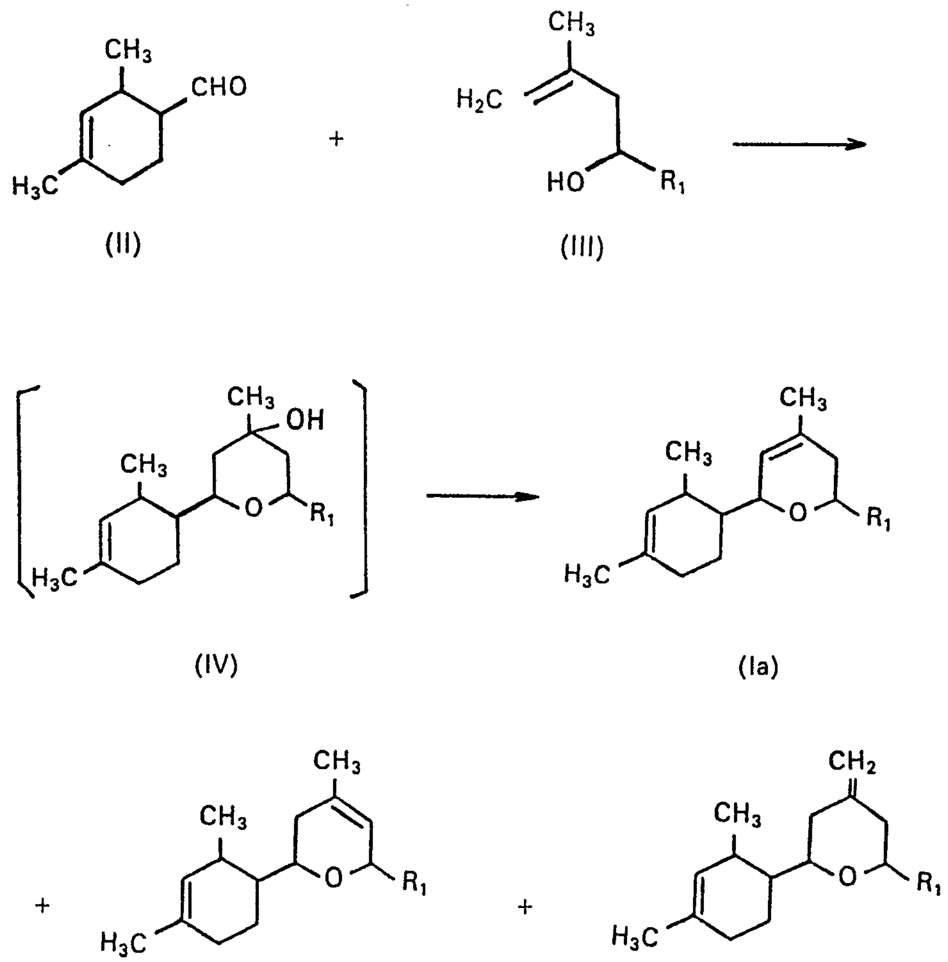

(II) (III)

(IV) (Ia)

(Ib) (Ic)

wherein $R_1$ is as defined above.

In the above process, although the molar ratio of the compound (III) to the compound (II) is not particularly defined, it is preferred to use 0.5 to 2.0 mol of the compound (III) per mol of the compound (II). A strong acid is preferably used as said acid catalyst, which includes, for example sulfuric, p-toluenesulfonic, hydrochloric, and phosphoric acids. The amount of the catalyst, though it depends on the kind of the catalyst, is preferably within the range of 0.01 to 10% based on the amount of the starting material.

The reaction is preferably carried out in a solvent which can azeotropically remove water from the reaction system, since the reaction involves dehydration. Hexane, benzene, toluene, xylene, and the like are preferably used as the solvent.

Since an intermediate (IV) is formed in the process of the present reaction as shown in the above reaction formula, the reaction can be effected either in one step or in two steps. The reaction is usually effected at a temperature ranging from 10 to 200 °C. If the reaction is effected in one step, the temperature is preferably within the range of 50 to 150 °C, more preferably from 60 to 120 °C. When the reaction is effected in two steps, it is preferred to conduct the first reaction at 10 to 100 °C and the second reaction at 50 to 150 °C. The reaction time is, though it depends on the reaction temperature, usually 1 to 200 hours.

The product is obtained in this way as an isomeric mixture of (Ia), (Ib), and (Ic).

As described heretofore, the compound (I) of the present invention is excellent in stability and has persistent green note perfume like tomato leaf. Accordingly, the present compound can be used for the preparation of various perfume compositions.

The present invention will now be described in more detail by way of the following examples.

Example 1

A mixture of 69 g (0.5 mol) of 2-methyl-1-penten-4-ol (72% purity), 69 g (0.5 mol) of 2,4-dimethyl- 3-cyclohexene-1-carboxaldehyde,

and 1 g of p-toluenesulfonic acid was stirred at room temperature for 96 hours. The mixture was then treated with 100 g of an aqueous sodium carbonate solution (5%) and washed with water three times. 100 g of xylene and 0.3 g of conc. sulfuric acid were added thereto, and the reaction was continued under reflux for 3 hours, the generated water being removed out of the reaction system by azeotropic distillation with xylene. The reaction product was treated with 100 g of an aqueous sodium carbonate solution (5%) and washed with water three times. After the solvent and the low-boiling fractions had been distilled off, the distillation was continued to obtain 79 g of a fraction at 110 to 120 °C at 3.5 mmHg. The obtained fraction was further subjected to precision distillation, yielding 35 g of a fraction at 106 to 108 °C at 3.5 mmHg. As the result of the determination of the product by gas chromatography using Carbowax 20 M (capillary column 50 m) and gas mass spectroscopy, it was recognized that the product was a mixture of 2,4-dimethyl-6-(2,4-dimethyl-3-cyclohexenyl)-3,6-dihydro-2H-pyran (Ia, $R_1$ = $CH_3$), 4,6-dimethyl-2-(2,4-dimethyl-3-cyclohexenyl)- 3,6-dihydro-2H-pyran (Ib, $R_1$ = $CH_3$), and 2-(2,4-dimethyl-3-cyclohexenyl)- 6-methyl- 4-methylenetetrahydropyran (Ic, $R_1$ = $CH_3$), which are the compounds of the formulae (Ia) to (Ic) wherein $R_1$ is $CH_3$, and that the purity of the product was at least 99%. The parent peak was found at m/e = 220. The results of the elementary analysis, $^1$H–NMR, and IR are as shown below, from which it was further recognized that the obtained product was a mixture of the above-mentioned three components.

Elementary analysis:

| | | | |
|---|---|---|---|
| calculated (%): | C 81.76, | H 10.98, | O 7.26 |
| found (%): | C 81.74, | H 10.99, | O 7.27 |

$^1$H–NMR ($CDCl_3$, δ 5.4 TMS as internal reference, δ): Figure 1

cyclohexene ring, 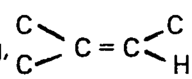 pyran

rings of (Ia–$CH_3$) and (Ib–$CH_3$),

composite peak of 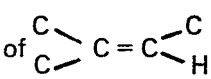

4.8 pyran ring of (Ic–$CH_3$), 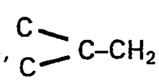

0.9–4.4 complex multiplet (22H)

IR ($cm^{-1}$): Figure 2 2960, 2930, 2900, 1675, 1650, 1440, 1375, 1320, 1220, 1170, 1100, 1055, 1025, 890, 860, 820, 805

Example 2

The reaction was effected in the same manner as in Example 1, except that 43 g of 2-methyl-1-buten-4-ol was used in place of 4-methyl-4-penten-2-ol. 28 g of a mixture of 6-(2,4-dimethyl-3-cyclohexenyl) -4-methyl-3,6-dihydro -2H-pyran (Ia, $R_1$ = H), 2-(2,4-dimethyl-3-cyclohexenyl)-4-methyl-3,6-dihydro-2H-pyran (Ib, $R_1$ = H), and 2-(2,4-dimethyl-3-cyclohexenyl)-4-methylenetetrahydropyran (Ic, $R_1$ = H) was obtained. The product was determined by gas chromatography using Carbowax 20 M (capillary column 50 m) and gas mass spectroscopy to reveal that it has a purity of 99% or more. The parent peak was found at m/e = 206.

Elementary analysis:

| | | | |
|---|---|---|---|
| calculated (%): | C 81.50, | H 10.75, | O 7.76 |
| found (%): | C 81.47, | H 10.74, | O 7.78 |

Example 3

A mixture of 238 g (1.7 mol) of 2,4-dimethyl-3-cyclohexene-1-carboxaldehyde, and 238 g (2.0 mol) of 2-methyl-1-penten-4-ol having a purity of 72% was dropped under reflux to a mixture of 180 g of hexane and 3 g of conc. sulfuric acid over a period of 1 hour. The water generated by the reaction was removed out of the reaction system by azeotropic distillation with hexane. The reaction was further continued for 8 hours under reflux after the completion of the dropping. The reaction product was washed with a 5% sodium carbonate solution and then with water three times. After the topping of hexane under a reduced pressure, the product was distilled to yield 318 g of a fraction at 100 to 120 °C at 3.5 mmHg. The fraction was subjected to precision distillation to yield 227 g of a fraction at 106 to 108 °C at 3.5 mmHg (61% yield). The results of the determination of the product by gas chromatography, gas mass sepctroscopy, elementary analysis, $^1$H–NMR, and IR were completely coincident with those of Example 1.

Example 4

| Rose oil-type perfume: | |
|---|---|
| geraniol | 250 parts |
| citronellal | 200 |
| rosinol | 100 |
| phenylethyl alcohol extract | 310 |
| nerol | 30 |
| geranium oil Africa | 10 |
| | 900 |

100 parts of the compound obtained in Example 1 was added to 900 parts of the rose oil having the above composition, whereby a fresh and clean new rose oil was obtained.

Example 5

| Bergamot oil-type perfume: | |
|---|---|
| linalyl acetate | 550 parts |
| linalool | 300 |
| limonene | 30 |
| citral | 20 |

| Bergamot oil-type perfume: | |
|---|---|
| nonyl aldehyde | 1 |
| decyl aldehyde | 2 |
| a-pinene | 5 |
| b-pinene | 5 |
| terpineol | 20 |
| coumarin | 1 |
| petitgrain paraguay | 5 |
| ocimene | 11 |
| | 950 |

50 parts of the compound obtained in Example 1 was added to 950 parts of the bergamot oil having the above composition, whereby more natural, sweet, and strong-scented bergamot oil was obtained.

Brief Description of the Drawings:

Figure 1 is a $^1H$–NMR spectrum of the product obtained in Example 1, and Figure 2 is an IR spectrum of the same product.

## Claims

1. Isomeric mixture of a pyran derivative represented by the general formula (I):

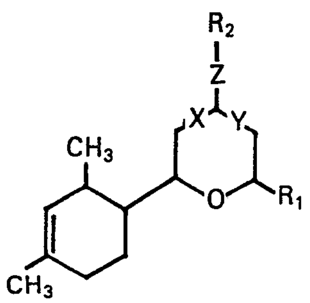

(I)

wherein $R_1$ stands for H or $CH_3$ and $R_2$ for $CH_2$ or $CH_3$, one of the bonds X, Y, and Z is a double bond and the other two are single bonds.

2. A perfume composition containing an isomeric mixture of a pyran derivative represented by the general formula (I):

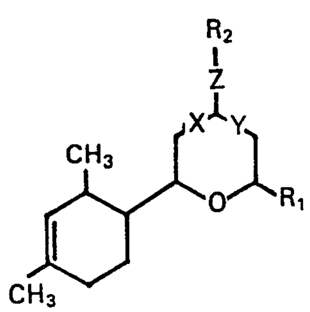

(I)

wherein $R_1$ stands for H or $CH_3$ and $R_2$ for $CH_2$ or $CH_3$, one of the bonds X, Y, and Z is a double bond and the other two are single bonds.

## Patentansprüche

1. Isomerisches Gemisch aus einem Pyranderivat der allgemeinen Formel (I):

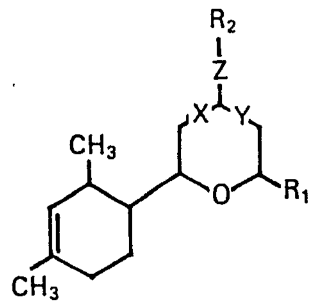

(I)

worin $R_1$ für H oder $CH_3$ steht und $R_2$ für $CH_2$ oder $CH_3$ steht, eine der Bindungen X, Y und Z eine Doppelbindung ist und die beiden anderen Einfachbindungen sind.

2. Parfumzusammensetzung, dadurch gekennzeichnet, dass sie ein isomerisches Gemisch aus einem Pyranderivat der allgemeinen Formel (I) enthält:

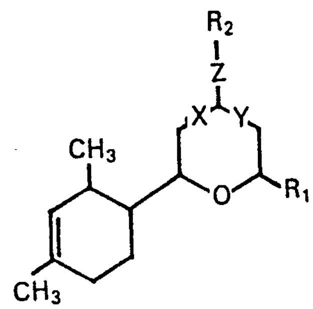

(I)

worin $R_1$ für H oder $CH_3$ steht und $R_2$ für $CH_2$ oder $CH_3$ steht, eine der Bindungen X, Y und Z eine Doppelbindung ist und die beiden anderen Einfachbindungen sind.

## Revendications

1. Mélange isomère d'un dérivé du pyranne représenté par la formule générale (I):

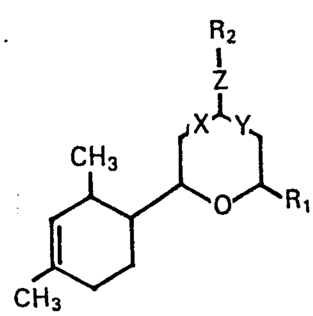

(I)

dans laquelle $R_1$ est un atome d'hydrogène ou un groupe $CH_3$ et $R_2$ est un radical $CH_2$ ou $CH_3$, l'une des liaisons X, Y et Z est une double liaison et les deux autres sont des liaisons simples.

2. Composition de parfum contenant un mélange isomère d'un dérivé du pyranne représenté par la formule générale (I):

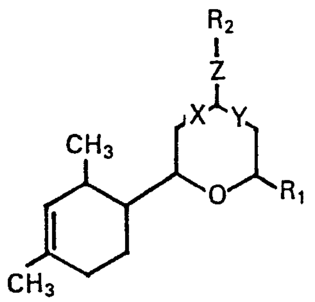

(I)

dans laquelle $R_1$ est un atome d'hydrogène ou un groupe $CH_3$ et $R_2$ est un radical $CH_2$ ou $CH_3$, l'une des liaisons X, Y et Z est une double liaison et les deux autres sont des liaisons simples.

1/2

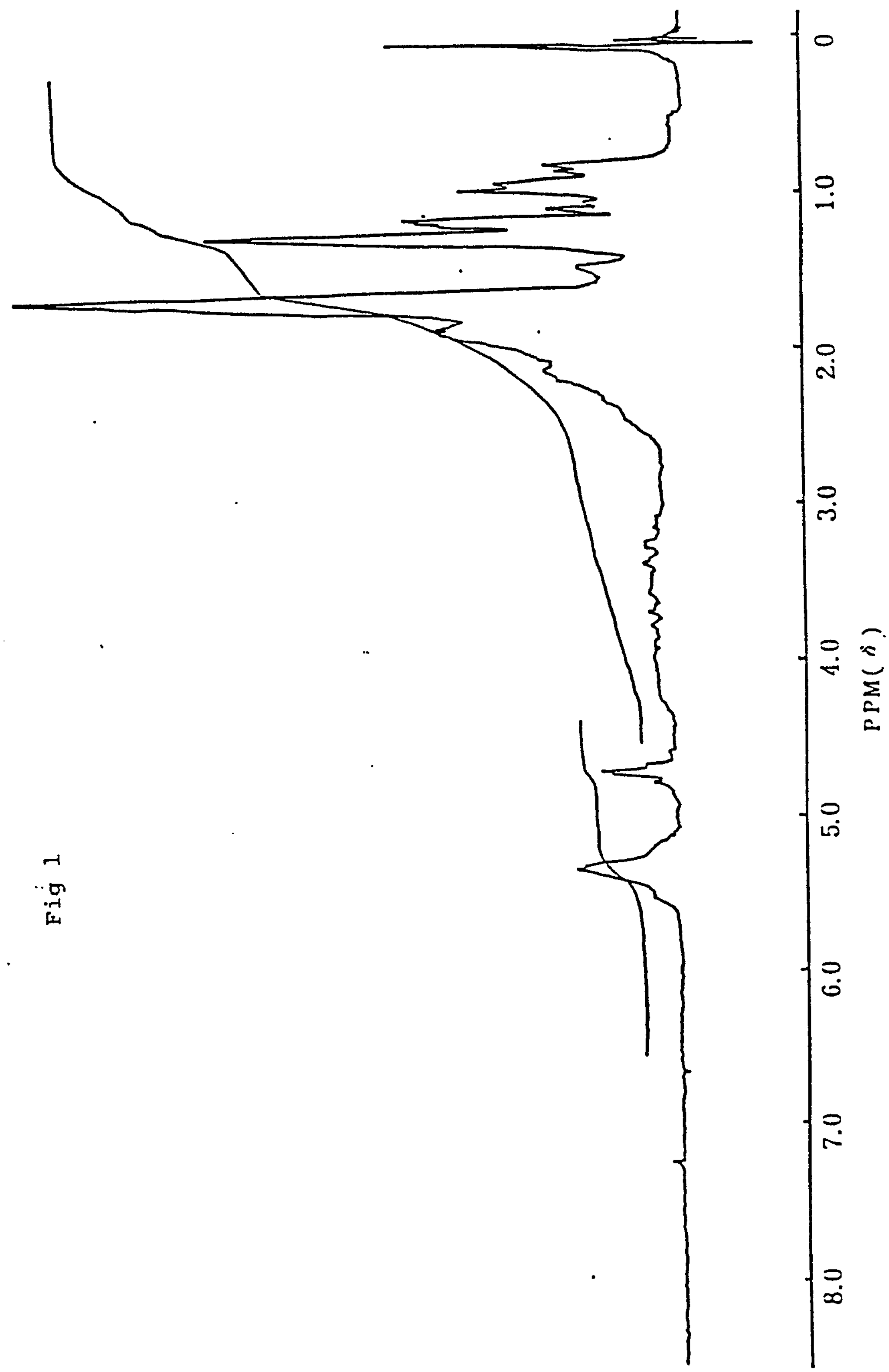


Fig 1

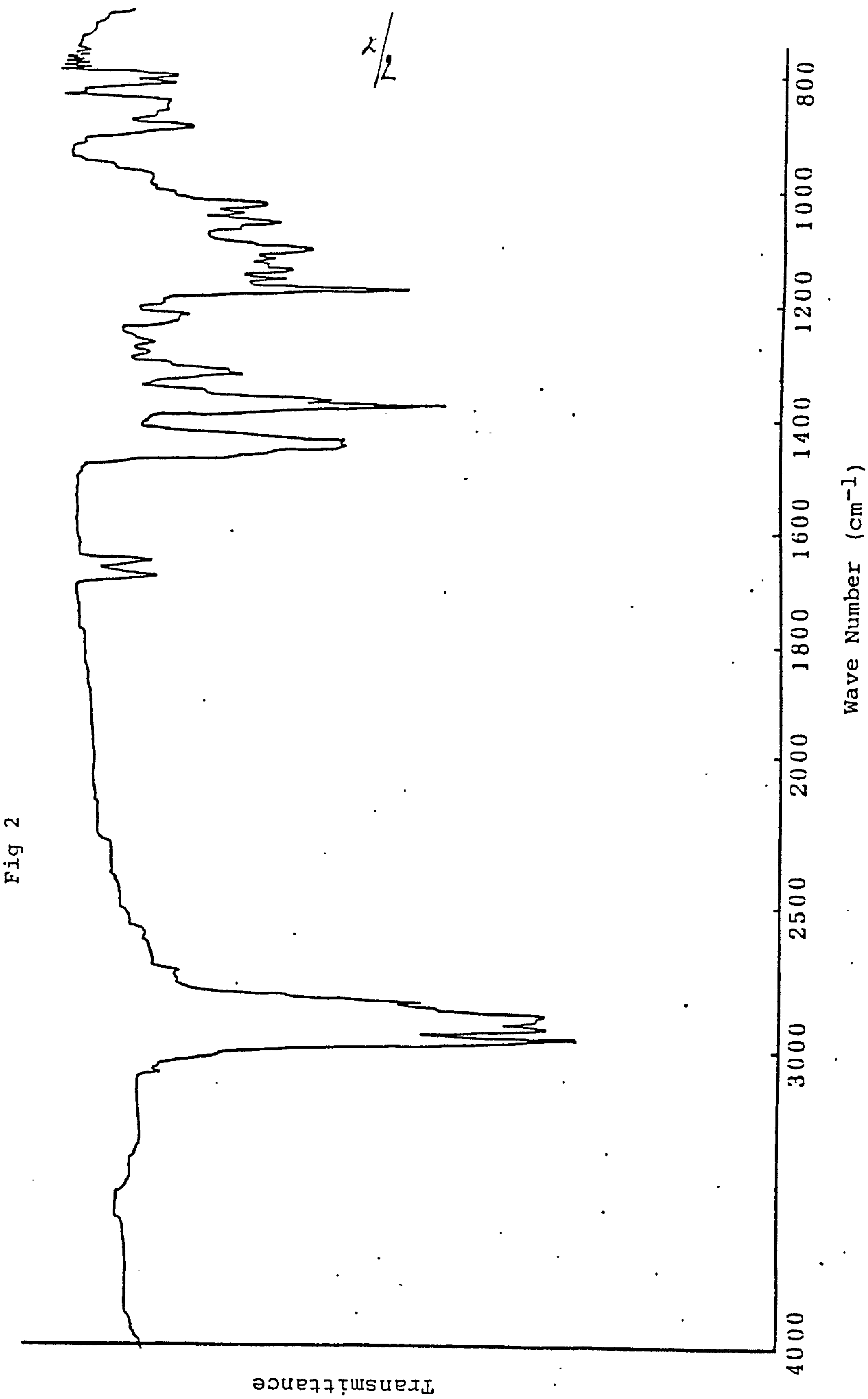
800
1000
1200
1400
1600
1800
2000
2500
3000
4000
Wave Number (cm$^{-1}$)
Transmittance

Fig 2